# EUROPEAN PATENT SPECIFICATION

(11) **EP 3 232 779 B1**
(45) Date of publication and mention of the grant of the patent: **17.09.2025**
(21) Application number: 15871266.1
(22) Date of filing: 21.12.2015
(51) Int. Cl.: A01N 1/147

(54) **A CLOSED SYSTEM CRYOPRESERVATION DEVICE**
KRYOKONSERVIERUNGSVORRICHTUNG MIT GESCHLOSSENEM SYSTEM
DISPOSITIF DE CRYOCONSERVATION EN SYSTÈME FERMÉ

(30) Priority: 19.12.2014 US 201414577578
(43) Date of publication of application: 25.10.2017
(73) Proprietor: Biotech, Inc., Alpharetta, GA 30005 (US)
(72) Inventor: PARRA, Jorge E., Cumming, GA 30040 (US); BERNAL, Diana P., Cumming, GA 30040 (US)
(74) Representative: dompatent von Kreisler Selting Werner - Partnerschaft von Patent- und Rechtsanwälten mbB
(86) International application number: PCT/US2015/066996
(87) International publication number: WO 2016/100962

(56) References cited:
- WO-A1-2017/099865
- US-A1- 2005 287 512
- US-A1- 2006 134 596
- US-A1- 2010 151 570
- US-A1- 2014 157 798
- US-A1- 2014 158 695
- US-B1- 6 337 205
- ANONYMOUS: "BIOTECH INC: CRYOLOCK(TM)", 1 January 2014 (2014-01-01), XP055505942, Retrieved from the Internet <URL:http://www.irvinesci.com/uploads/technical-documentations/Cryolock_Semi-closed_System_Brochure_Jan-2014.pdf> [retrieved on 20180910]
- A. STEIN ET AL: "Successful use of the Cryolock device for cryopreservation of scarce human ejaculate and testicular spermatozoa", ANDROLOGY, vol. 3, no. 2, 5 February 2015 (2015-02-05), pages 220 - 224, XP055505948, ISSN: 2047-2919, DOI: 10.1111/andr.12007
- ANONYMOUS: "Cryolock(TM) (archived webpage)", 16 December 2014 (2014-12-16), XP055506011, Retrieved from the Internet <URL:https://web.archive.org/web/20141216224023/http://cryolock.info/cryolock/index.php> [retrieved on 20180911]

## Description

### BACKGROUND

### Field

The device described and claimed herein is in the field of devices for the cryopreservation of biological specimens.

### Description of the Problem and Related Art

Cryopreservation is practiced in the life sciences for the purpose of halting biological activity in valuable cells for an extended period of time. Among the techniques used for cryopreservation is vitrification.

Vitrification involves the transformation of a solution comprised of a biological specimen, i.e., an oocyte or an embryo, into a glass-like amorphous solid that is free from any crystalline structure, followed by extremely rapid cooling. One of the major challenges of this method is to prevent the intracellular liquid within the oocyte or embryo to form ice crystals. Accordingly, the first step is to dehydrate the cell or cells as much as possible using cryoprotectant containing fluids called "vitrification media." The biological specimen is then rapidly chilled by immersion in a cryogenic fluid such as liquid nitrogen (LN₂). With a proper combination of chilling speed and cryoprotectant concentration, intracellular water will attain a solid, innocuous, glassy (vitreous) state rather than an orderly, damaging, crystalline ice state. Vitrification can be described as a rapid increase in fluid viscosity that traps the water molecules in a random orientation. Vitrification media, however, can contain relatively high levels of cryoprotectant that can be toxic to cells except in the vitreous state. As a result, the time exposure of cells to vitrification media during dehydration and warming must be carefully controlled to avoid cellular injury, and, accordingly, it is desirable to chill the specimen as quickly as possible.

This impetus led to development of a method in which the biological specimen is directly immersed in the cryogen to achieve rapid chilling. Cryocontainer devices used in this technique are classified as "open" for use in an "open system" because the biological specimen is in direct contact with the cryogen, e.g., LN₂. Examples include electron microscopy grids, open pulled straws, the Cryoloop^{™}, from Hampton Research Corp., of Aliso Viejo, California, USA, and Cryotop^{®} offered by KitaZato Biopharma Co. Ltd, of Fuji, Shizuoka, Japan. Open carriers also enable rapid warming of the biological specimen.

LN₂, however, is not aseptic. It may contain bacterial and fungal species, which are viable upon warming. Furthermore, it has been reported that vitrified cells held in long term storage in LN₂ could be infected by viral pathogens artificially placed in said LN₂. Hence, there is the potential for infection of biological samples vitrified in open carriers. As a result, many countries have banned open systems due to the high risk of sample contamination.

The potential of infection has led to the development of sealed cryocontainers where the biological sample is placed in a cryocontainer and sealed before chilling in LN₂. The cryocontainer also serves as a storage device to isolate it from the cryogen during long-term storage. A "closed" system refers to a vitrification system that prevents direct contact between LN₂ and the biological material. Examples of closed cryocontainers include Cryotip^{®}, offered by Irvine Scientific, and the "Cryotop^{®} SC" from KitaZato. In both cases, the containers are heat-sealed to enclose the specimen.

Another example of a cryocontainer device for use in closed system, U.S. Pat. No. 7,316,896, to Kuwayama, et al.*,* "Egg freezing and storing tool and method", describes a closed cryocontainer for vitrification. This device comprises a fine plastic tube (nominally 0.25 mm OD and a wall thickness of 0.02 mm). A typical biological specimen will contain a human oocyte having an OD of 0.125 mm. It is dehydrated with vitrification media and then drawn into the tube. Then both ends of the tube are heat-sealed with a thermal sealing device to create an aseptic container. Because one of the heat seals is created very close to the biological specimen, there are concerns that the heat will injure the cell.

Similarly, U.S. Pat. No. 8,372,633, "Kit for Packaging Predetermined Volume of Substance to be Preserved by Cryogenic Vitrification", to Clairaz, et al.*,* describes a tube-within-a-tube closed cryocontainer concept. Both tubes are fabricated from plastic. The inner tube is modified to create a channel at one end upon which the biological specimen is placed. The loaded inner tube is then placed within the outer tube. The outer tube is then heat-sealed at the loading end to create an aseptic cryocontainer. However, heat-sealing requires a costly sealing device capable of fusing the plastic of a vitrification cryocontainer. It also adds another step in a process that requires speed for safe execution.

To address this short-coming, a closed system container device is presented by U.S. Pub. App. 20090123996, by Chin*,* and entitled, "Vitrification Device With Shape Memory Seal." The device is disclosed to comprises a specimen collection tube in one end of which a stopper is installed. The collection tube, with specimen, is inserted into a tubular sheath until the stopper engages the sheath. Then, instead of heat-sealing, a separate closing device is installed on the stoppered sheath. The closing device comprises a cap that is drawn down on the stopper by a shape memory material that contracts when subjected to low temperatures, such as when immersed in LN₂. A problem with this approach is that it increases the complexity of the cryocontainer device, and because the components are made of different materials, each having its own coefficient of thermal expansion, they expand or contract at different rates which may also disrupt the seal.

US 2014/ 157798 A1 discloses a cryogenic storage device, such as a cryobath, for the cryopreservation and/or vitrification of a biological sample in a freezing medium. A vial that may be used with the cryogenic storage device is also described, which comprises a closure member that may be removably received by the open end of the vial. The closure member may comprise a deformable sealing member that provides a shaped male taper which tightly and securely engages a corresponding female taper of the internal lumen wall of the vial, such as by friction fit, in order to provide a leakproof impermeable seal. In other words, a "closed system" within the vial lumen may be created and maintained for sealed storage of a sample in a variety of environments and temperature ranges, including during vitrification and storage in liquid nitrogen, without the need for providing additional or supplemental sealing measures to protect the samples during storage and/or to prevent liquid nitrogen from penetrating into the specimen-retaining chamber of the vial. The vial and the closure member are preferably constructed of the same material so as to ensure the same thermal contraction properties of the respective materials.

The successful use of the Cryolock device for cryopreservation of scarce human ejaculate and testicular spermatozoa is discloses in Andrology, 2015, 3, p. 220-224 (A. Stein et al.).

### BRIEF DESCRIPTION OF THE DRAWINGS

The present device is described with reference to the accompanying drawings. In the drawings, like reference numbers indicate identical or functionally similar elements.
FIG. 1 is a side view of an exemplary embodiment of the disclosed cryopreservation device;
FIG. 2A illustrates a stick member of
FIG. 2B illustrates a cap for the embodiment shown in FIG. 1;
FIG. 3A is a detailed view of one end of the stick member;
FIG. 3B a detailed view of the end of the stick member shown in FIG. 3A, with the stick member rotated 90° about the long axis;
FIG. 4 is a detailed, fragmented view illustrating the cap engaged on the stick member; and
FIGs. 5A through 5D show various exemplary shapes that may comprise the perpendicular cross-section of the device.

### DETAILED DESCRIPTION

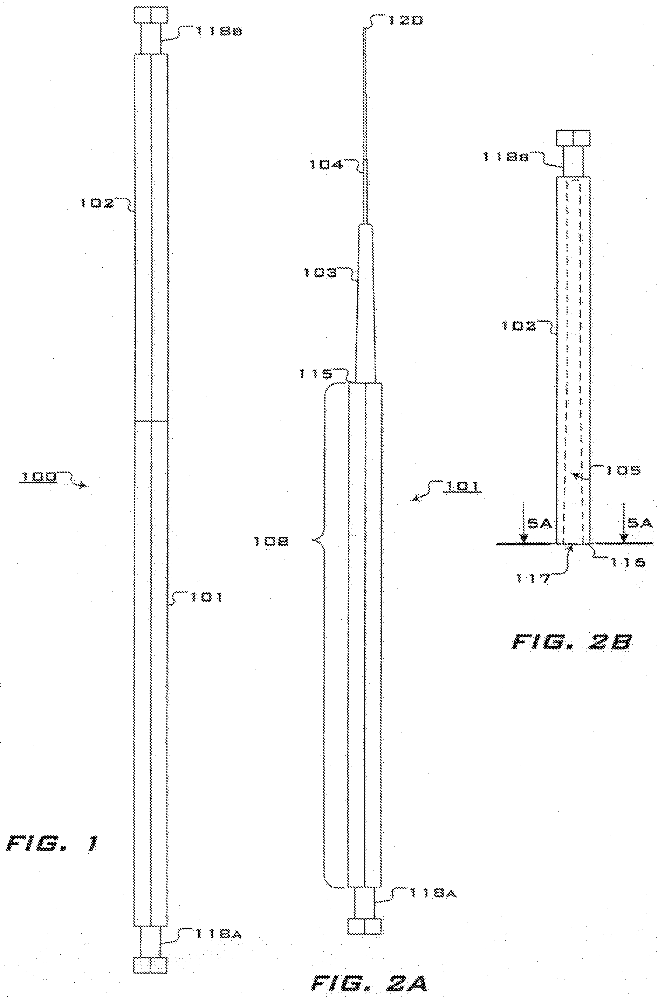

The present invention therefore provides a closed system cryopreservation device as claimed in claim 1, with preferred embodiments being claimed in the dependent claims. The various embodiments of the closed system cryopreservation storage device describe below and their advantages are best understood by referring to FIGs. 1 through 5D of the drawings. The elements of the drawings are not necessarily to scale, emphasis instead being placed upon clearly illustrating the novel features and principles of operation. Throughout the drawings, like numerals are used for like and corresponding parts of the various drawings.

Furthermore, reference in the specification to "an embodiment," "one embodiment," "various embodiments," or any variant thereof means that a particular feature or aspect described in conjunction with the particular embodiment is included in at least one embodiment. Thus, the appearance of the phrases "in one embodiment," "in another embodiment," or variations thereof in various places throughout the specification are not necessarily all referring to its respective embodiment.

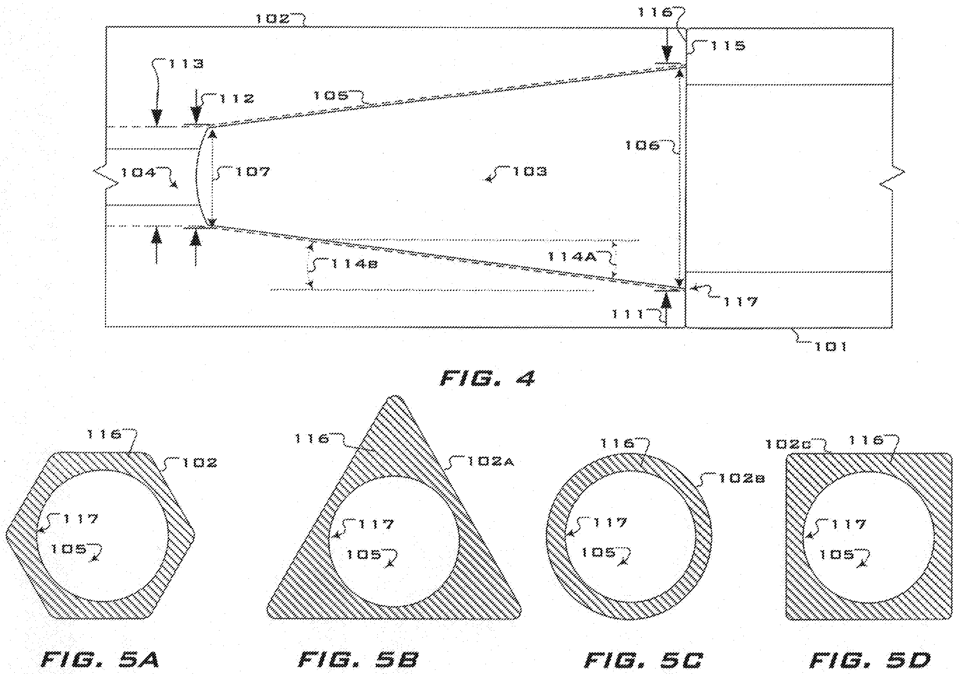

Referring to FIGs. 1, 2A & 2B, a closed system cryopreservation device 100 comprises an elongated stick 101 and a cap 102. The stick comprises a body 108 having a generally uniform cross-section abruptly transitioning at roughly midway along the stick 101 to a frustoconical boss 103. A shoulder 115 having a generally planar surface oriented roughly perpendicularly to the long axis of the stick 101 is formed at the transition from the body 108 to the boss 103. A specimen collection tip 104 extends from the narrow end of the boss 103. The cap 102 comprises an open end 116 having a generally planar surface in which is defined a circular opening 117. The opening 117 is in communication with an elongated hollow chamber 105 defined along the long axis of the cap 102 and dimensioned to accommodate the tip 104 and the boss 103. The cap 102 preferably comprises the same cross-sectional shape as the stick 101, e.g., hexagonal (FIG. 5A), triangular (FIG. 5B), square (FIG. 5C, circular (FIG. 5D), or the like, and roughly equal cross-sectional dimensions. Additionally, an optional, advantageous structural feature is a circumferential notch 118a, b disposed near the ends of the stick 101 and the cap 102, respectively, by which the device 100 may be clasped with forceps, making the device 100 easier to hold the device 100.

When a specimen (oocyte or embryo) is to be vitrified, it is collected and processed according to, for example, the protocol described above, and then deposited on the specimen collection tip 104. The tip 104 is then inserted into the elongated chamber 105 through the opening 117 and the cap 102 is pressed into place, until the planar surface of the open end 116 is seated against the planar surface of the shoulder 115. Accordingly, it will be appreciated that one planar surface should be substantially parallel with the opposing planar surface.

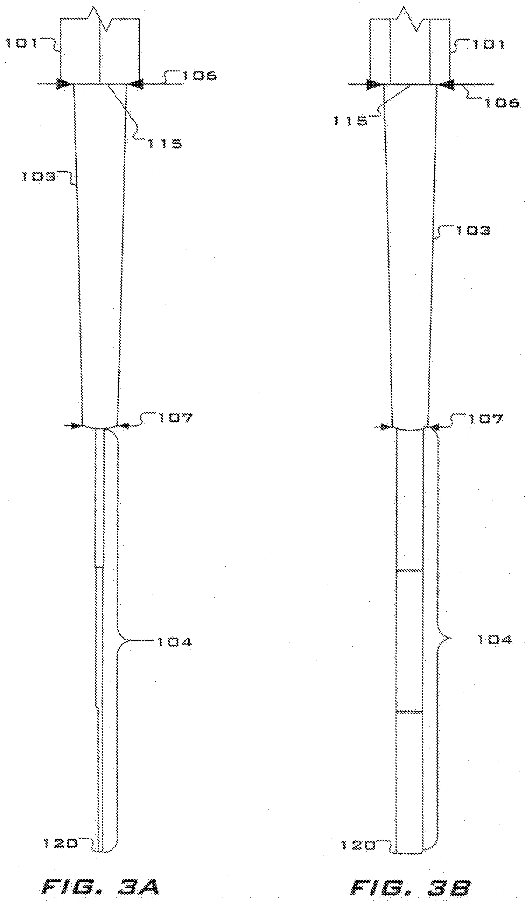

FIGs. 3A & 3B are detailed views, one rotated 90° from the other, of the portion of the stick 101 comprising the boss 103 and the tip 104. The boss 103 comprises a first outside diameter 106 (O.D.) at the base of the frustum adjacent the shoulder 115, and a second O.D. 107 at the distal end of the boss 103.

Referring now to FIG. 4, the cap 102 is shown seated against the shoulder 115 of the stick member 101 such that the tip 104 and the boss 103 are housed within the hollow chamber 105. It will be appreciated that the proportions illustrated in this view are exaggerated and not to scale to clearly show the dimensional features of the cap 102 and boss 103 and the inter-engagement of the two pieces. Accordingly, the proportions or dimensions that may be suggested in FIG. 4 are not to be construed as limiting any dimension to a particular value unless expressly defined herein.

As shown, boss 103 includes a first O.D. 106 that is greater than a second O.D. 107, the diameter of the boss tapering from the first O.D. 106 to the second O.D. 107 according to an angle 114A. The hollow chamber 105 comprises a first section defined from the open end 116 of the cap 102 and which is configured with a first inside diameter 111 (I.D.) located at the opening 117 and a second I.D. 112, such that the first I.D. 111 is greater than the second I.D. 112, decreasing according to angle 1114B. The second section of the chamber 105 comprises an elongated portion having a third I.D. 113 that is dimensioned to accommodate the tip 104. Therefore, the interior surface of the first section of the hollow chamber 105 defines a frustoconical space.

In this embodiment, angles 114A and 114B are roughly equal, preferably within a tolerance of 0.1%. The degree of taper should be relatively slight, no more than about 1.5°, and preferably about .50°. Thus, the first I.D. 111 is greater than the first O.D. 106, and the second I.D. 112 is greater than the second O.D. 107, in both cases by no more than about 0.1%. Accordingly, the frustoconical space defined by the interior surface of the hollow chamber 105 corresponds to the volume of the frustoconical boss 103 such that when the cap 102 is seated on the body 108, the specimen collection tip 104 is enclosed within the second section of the hollow chamber 105 and substantially all of the interior surface of the frustoconical section of the chamber 105 is in contact with the exterior surface of the boss 103. In this way, the hollow chamber 105 is sealed against entry of LN₂ when the cap 102 is properly installed without taking an additional step of heat-sealing the device.

Furthermore, there is also no need for a gasket. Typically such gaskets are comprised or a flexible, resilient material suitable for use with LN₂, such as silicon. However, silicon possesses a coefficient of thermal expansion different from the rigid material used to form the body and the cap.

On the other hand, the stick 101 and the cap 102 are made of the same rigid material which is suitable for immersion in cryogenic substances so that both pieces exhibit the same coefficient of thermal expansion. Various polymers may be used: polyester (for example, polyethylene terephthalate, polybutylene terephthalate); polyolefin (for example, polyethylene, ultra-high molecular-weight polyethylene, polypropylene, ethylene-propylene copolymer, ethylene-vinyl acetate copolymer), styrene resin (for example, polystyrene, methacrylate-styrene copolymer, methacrylate-butylene-styrene copolymer); and polyamide (for example, nylon 6, nylon 66). Preferably, both the stick 101 and the cap 102 are formed from a medical grade polystyrene crystal. Thus, the volumes of both pieces expand or contract in response temperature at the same rate insuring the interior surface of the frustoconical portion of the hollow chamber remains in substantially full contact with the exterior surface of the boss 103, maintaining the seal provided by the cap. Thus, the device maintains an equally secure seal both at room temperature and at low cryogenic temperatures, facilitating substantially uniform temperature conduction throughout the entire volume of the device.

As described above and shown in the associated drawings, the present invention comprises a closed system cryopreservation device. While particular embodiments have been described, it will be understood, however, that the present invention is not limited thereto, since modifications may be made within the scope of the appended claims by those skilled in the art, particularly in light of the foregoing teachings.

## Claims

1. A closed system cryopreservation device (100) for vitrification of biological specimens, said cryopreservation device (100) comprising:
an elongated body (108) comprising a first long axis and a perpendicular cross-section shape;
a frustoconical boss (103) having an exterior surface and extending from a first end of said elongated body (108), wherein a shoulder (115) having a first planar surface oriented perpendicularly to said first long axis is formed at the transition from said body (108) to said boss (103);
a specimen collection tip (104) extending from said boss (103); and
an elongated cap (102) for sealably enclosing said specimen collection tip (104), said cap (102) comprising a second long axis and a perpendicular cross-section shape, said cap (102) having a first end (116) that has a second planar surface oriented perpendicularly to said second long axis, an opening (117) being defined in said second planar surface, the opening (117) being in communication with an elongated hollow chamber (105) extending along said second long axis and having a length sufficient to accommodate said tip (104) and said boss (103), said hollow chamber (105) comprising a first section and a second section, wherein the first section is defined from the first end (116) of the cap (102) and is configured with a first inside diameter (111) located at the opening (117) of the first end (116) and a second inside diameter (112) at the opposite end of the first section, the first inside diameter (111) being greater than
the second inside diameter (112), said second section corresponding to said specimen collection tip (104), the second section comprising an elongated portion having a third inside diameter (113) that is dimensioned to accommodate said specimen collection tip (104), wherein said hollow chamber (105) comprises a frustoconical interior surface at said first section that defines a frustoconical space with a volume corresponding to said frustoconical boss (103), such that when said boss (103) is inserted into said hollow chamber (105), said boss (103) fills substantially all of the frustoconical space of said first section of said hollow chamber (105) when said second planar surface is seated against said first planar surface and substantially all of said interior surface is in contact with said exterior surface of said boss (103), wherein said frustoconical boss (103) comprises an outside diameter (106, 107) and said frustoconical space comprises an inside diameter (111, 112, 113), and wherein said inside diameter (111, 112, 113) is greater than said outside diameter (106, 107) at all points but by no more than about 0.1%;
wherein said device (100) comprises a substantially uniform coefficient of thermal expansion; wherein the device (100) further comprises a first angle (114A) defined by a decrease in diameter (106, 107) of said frustoconical boss (103) and a second angle (114B) defined by a decrease in diameter (111, 112, 113) of said frustoconical space, wherein said first and second angles (114A, 114B) are substantially equal, and wherein said first and second angles (114A, 114B) are no greater than about 1.5°.

2. The closed system cryopreservation device (100) of Claim 1, further comprising at least one circumferential notch (118a, 118b) defined in either of said body (108) or said cap (102).

3. The closed system cryopreservation device (100) of Claim 1, wherein said cross-section shape of said body (108) is one of circular, rectangular, triangular, and hexagonal.

4. The closed system cryopreservation device (100) of Claim 1, wherein said body (108) and said cap (102) comprise the same material.

5. The closed system cryopreservation device (100) of Claim 4, wherein both the stick (101), comprising the elongated body (108), the frustoconical boss (103) and the specimen collection tip (104), and the cap (102) comprise a crystal polystyrene.

6. The closed system cryopreservation device (100) of Claim 1, wherein said first and second angles (114A, 114B) are about 0.5°.

## Patentansprüche

1. Eine geschlossene Kryokonservierungsvorrichtung (100) zur Vitrifizierung biologischer Proben, wobei die Kryokonservierungsvorrichtung (100) umfasst:
einen länglichen Korpus (108), der einer ersten Längsachse und eine dazu senkrechte Querschnittsform umfasst;
einen kegelstumpfförmigen Vorsprung (103), der eine Außenfläche aufweist und sich von einem ersten Ende des länglichen Korpus (108) aus erstreckt, wobei am Übergang von dem Korpus (108) zu dem Vorsprung (103) eine Schulter (115) mit einer ersten planen Fläche, die senkrecht zu der ersten Längsachse ausgerichtet ist, ausgebildet ist;
eine Probensammelspitze (104), die sich von dem Vorsprung (103) aus erstreckt; und
eine längliche Verschlusskappe (102) zum dichten Verschließen der Probensammelspitze (104), wobei die Verschlusskappe (102) eine zweite Längsachse und eine dazu senkrechte Querschnittsform umfasst, die Verschlusskappe (102) ein erstes Ende (116) aufweist, das eine zweite plane Fläche aufweist, die senkrecht zu der zweiten Längsachse ausgerichtet ist, wobei in der zweiten planen Fläche eine Öffnung (117) definiert ist, wobei die Öffnung (117) mit einer länglichen Hohlkammer (105) in Verbindung steht, die sich entlang der zweiten Längsachse erstreckt und eine ausreichende Länge hat, um die Spitze (104) und den Vorsprung (103) aufzunehmen, wobei die Hohlkammer (105) einen ersten Abschnitt und einen zweiten Abschnitt umfasst, wobei der erste Abschnitt von dem ersten Ende (116) der Verschlusskappe (102) definiert ist und mit einem ersten Innendurchmesser (111), der sich an der Öffnung (117) des ersten Endes (116) befindet, und einem zweiten Innendurchmesser (112) am gegenüberliegenden Ende des ersten Abschnitts konfiguriert ist, wobei der erste Innendurchmesser (111) größer ist als der zweite Innendurchmesser (112), wobei der zweite Abschnitt der Probensammelspitze (104) entspricht, wobei der zweite Abschnitt einen länglichen Teil umfasst, der einen dritten Innendurchmesser (113) aufweist, welcher so bemessen ist, dass er die Probensammelspitze (104) aufnehmen kann, wobei die Hohlkammer (105) an dem ersten Abschnitt eine kegelstumpfförmige Innenfläche umfasst, die einen kegelstumpfförmigen Raum mit einem Volumen, das dem kegelstumpfförmigen Vorsprung (103) entspricht, definiert, so dass dann, wenn der Vorsprung (103) in die Hohlkammer (105) eingeführt wird, der Vorsprung (103) im Wesentlichen den gesamten kegelstumpfförmigen Raum des ersten Abschnitts der Hohlkammer (105) ausfüllt, wenn die zweite plane Fläche auf der ersten planen Fläche anliegt und sich im Wesentlichen die gesamte Innenfläche in Kontakt mit der Außenfläche des Vorsprungs (103) befindet, wobei der kegelstumpfförmige Vorsprung (103) einen Außendurchmesser (106, 107) umfasst und der kegelstumpfförmige Raum einen Innendurchmesser (111, 112, 113) umfasst und wobei der Innendurchmesser (111, 112, 113) an allen Punkten um nicht mehr als etwa 0,1% größer ist als der Außendurchmesser (106, 107),
wobei die Vorrichtung (100) einen im Wesentlichen gleichmäßigen Wärmeausdehnungskoeffizienten umfasst; wobei die Vorrichtung (100) weiterhin einen ersten Winkel (114A), der durch eine Abnahme des Durchmessers (106, 107) des kegelstumpfförmigen Vorsprungs (103) definiert ist, und einen zweiten Winkel (114B), der durch eine Abnahme des Durchmessers (111, 112, 113) des kegelstumpfförmigen Raums definiert ist, umfasst, wobei der ersten und der zweite Winkel (114A, 114B) im Wesentlichen gleich sind und wobei der ersten und der zweite Winkel (114A, 114B) nicht größer als etwa 1,5° sind.

2. Die geschlossene Kryokonservierungsvorrichtung (100) gemäß Anspruch 1, weiterhin umfassend wenigstens eine umlaufende Kerbe (118a, 118b), die entweder in dem Korpus (108) oder in der Verschlusskappe (102) definiert ist.

3. Die geschlossene Kryokonservierungsvorrichtung (100) gemäß Anspruch 1, wobei die Querschnittsform des Korpus (108) kreisförmig, rechteckig, dreieckig oder sechseckig ist.

4. Die geschlossene Kryokonservierungsvorrichtung (100) gemäß Anspruch 1, wobei der Korpus (108) und die Verschlusskappe (102) dasselbe Material umfassen.

5. Die geschlossene Kryokonservierungsvorrichtung (100) gemäß Anspruch 4, wobei sowohl der Stift, den länglichen Korpus (108), den kegelstumpfförmigen Vorsprung (103) und die Probensammelspitze (104) umfasst, als auch die Verschlusskappe (102) ein kristallines Polystyrol umfassen.

6. Die geschlossene Kryokonservierungsvorrichtung (100) gemäß Anspruch 1, wobei der erste und der zweite Winkel (114A, 114B) etwa 0,5° betragen.

## Revendications

1. Un dispositif de cryoconservation en système fermé (100) pour la vitrification d'échantillons biologiques, ledit dispositif de cryoconservation (100) comprenant :
un corps allongé (108) comprenant un premier axe longitudinal et une forme de section transversale perpendiculaire ;
un bossage tronconique (103) ayant une surface extérieure et s'étendant à partir d'une première extrémité dudit corps allongé (108), dans lequel un épaulement (115) ayant une première surface plane orientée perpendiculairement audit premier axe longitudinal est formé à la transition dudit corps (108) vers ledit bossage (103) ;
une pointe de prélèvement d'échantillon (104) s'étendant à partir dudit bossage (103) ; et
un capuchon allongé (102) destiné à enfermer de manière étanche ladite pointe de prélèvement (104), ledit capuchon (102) comprenant un second axe longitudinal et une section transversale perpendiculaire, ledit capuchon (102) présentant une première extrémité (116) présentant une seconde surface plane orientée perpendiculairement audit second axe longitudinal, une ouverture (117) étant ménagée dans ladite seconde surface plane, ladite ouverture (117) communiquant avec une chambre creuse allongée (105) s'étendant le long dudit second axe longidutinal et d'une longueur suffisante pour loger ladite pointe (104) et ledit bossage (103), ladite chambre creuse (105) comprenant une première et une seconde section, la première section étant définie à partir de la première extrémité (116) du capuchon (102) et présentant un premier diamètre intérieur (111) situé au niveau de l'ouverture (117) de la première extrémité (116) et un second diamètre intérieur (112) à l'extrémité opposée de la première section, le premier diamètre intérieur (111) étant supérieur au deuxième diamètre intérieur (112), ladite deuxième section correspondant à ladite pointe de prélèvement d'échantillon (104), la deuxième section comprenant une partie allongée ayant un troisième diamètre intérieur (113) dimensionné pour accueillir ladite pointe de prélèvement d'échantillon (104), dans laquelle ladite chambre creuse (105) comprend une surface intérieure tronconique au niveau de ladite première section qui définit un espace tronconique avec un volume correspondant audit bossage tronconique (103), de telle sorte que lorsque ledit bossage (103) est inséré dans ladite chambre creuse (105), ledit bossage (103) remplisse sensiblement tout l'espace tronconique de ladite première section de ladite chambre creuse (105) lorsque ladite seconde surface plane est placée contre ladite première surface plane, et que sensiblement toute ladite surface intérieure soit en contact avec ladite surface extérieure dudit bossage (103), dans lequel ledit bossage tronconique (103) comprend un diamètre extérieur (106, 107) et ledit espace tronconique comprend un diamètre intérieur (111, 112, 113), et dans lequel ledit diamètre intérieur (111, 112, 113) est supérieur audit diamètre extérieur (106, 107) en tous points mais d'au plus environ 0,1 % ;
dans lequel ledit dispositif (100) comprend un coefficient de dilatation thermique sensiblement uniforme ; dans lequel le dispositif (100) comprend en outre un premier angle (114A) défini par une diminution du diamètre (106, 107) dudit bossage tronconique (103) et un deuxième angle (114B) défini par une diminution du diamètre (111, 112, 113) dudit espace tronconique, dans lequel lesdits premier et deuxième angles (114A, 114B) sont sensiblement égaux, et dans lequel lesdits premier et deuxième angles (114A, 114B) ne sont pas supérieurs à environ 1,5°.

2. Dispositif de cryoconservation à système fermé (100) selon la revendication 1, comprenant en outre au moins une encoche circonférentielle (118a, 118b) définie soit dans ledit corps (108), soit dans ledit capuchon (102).

3. Dispositif de cryoconservation à système fermé (100) selon la revendication 1, dans lequel ladite forme de section transversale dudit corps (108) est l'une des formes circulaire, rectangulaire, triangulaire et hexagonale.

4. Dispositif de cryoconservation à système fermé (100) selon la revendication 1, dans lequel ledit corps (108) et ledit capuchon (102) comprennent le même matériau.

5. Dispositif de cryoconservation à système fermé (100) selon la revendication 4, dans lequel à la fois le bâton (101), comprenant le corps allongé (108), le bossage tronconique (103) et la pointe de prélèvement d'échantillon (104), et le capuchon (102) comprennent un polystyrène cristallin.

6. Dispositif de cryoconservation à système fermé (100) selon la revendication 1, dans lequel lesdits premier et deuxième angles (114A, 114B) sont d'environ 0,5°.
